# EUROPEAN PATENT SPECIFICATION

(11) **EP 2 808 040 B1**
(45) Date of publication and mention of the grant of the patent: **17.05.2017**
(21) Application number: 13741436.3
(22) Date of filing: 25.01.2013
(51) Int. Cl.: A61L 27/34, A61L 27/54, A61L 31/10, A61L 31/16, C23C 18/02, C09D 183/04

(54) **OSTEOINDUCTIVE COATINGS FOR DENTAL IMPLANTS**
OSTEOINDUKTIVE BESCHICHTUNGEN FÜR ZAHNIMPLANTATE
REVÊTEMENTS OSTÉO-INDUCTEURS POUR IMPLANTS DENTAIRES

(30) Priority: 25.01.2012 ES 201230099
(43) Date of publication of application: 03.12.2014
(73) Proprietor: Universitat Jaume I De Castellón, 12006 Castellón de la Plana (ES); Universidad Del Pais Vasco Euskal Herriko Unibertsitatea, 48940 Leioa (Vizcaya) (ES)
(72) Inventor: SUAY ANTON, Julio José, 12006 Castellón De La Plana (ES); HERNÁNDEZ ESCOLANO, Miriam, 12006 Castellón De La Plana (ES); GOÑI ECHAVE, Isabel, 48940 Leioa (Vizcaya) (ES); GURRUCHAGA TORRECILLA, María Dolores, 48940 Leioa (Vizcaya) (ES)
(74) Representative: Ungria López, Javier
(86) International application number: PCT/ES2013/070031
(87) International publication number: WO 2013/110843

(56) References cited:
- WO-A1-2012/080544
- ES-A1- 2 384 762
- GB-A- 2 462 883
- GB-A- 2 462 883
- US-A1- 2007 071 789

## Description

### Technical field

The present invention relates to the field of osteoinductive coatings for dental implants of organic-inorganic hybrid nature, obtained by means of sol-gel technology.

### State of the art prior to the invention

In the field of maxillofacial surgery, implants have the function of replacing the tooth root, thereby creating a solid piece where the dental prosthesis or crown can be placed.

The use of dental implants has spread significantly in the last few years. It is estimated that the number of implants carried out each year is one million worldwide. However, there are patients that cannot be intervened due to several causes, such as patients with metabolic, haematological, or heart disease, or bone metabolism disorders. The capacity to regenerate bone of these patients is depleted, due to which the osseointegration process of the implant is compromised and dentists discourage the intervention. Other problems resulting in the failure of the implant are a deficit in bone quality and/or quantity, such as in elderly women or in patients who smoke (high risk factor for implant failure) [Implates, S.E.d., Libro Blanco de la Implantología Dental en España, 2008, Madrid: Sociedad Española de Implantes].

The most widely used materials have a metallic nature, pure titanium being the most consumed. In this field of surgical implantology, the purity of the material and the shape and topography of the implant can vary, as do the applied surface treatments. The purpose is to obtain a good mechanical connection to the bone in all cases, such that the movements that can take place between the implant and the peri-implant bone are reduced to the minimum with the final purpose of reducing the foreign body reaction [Spiekermann, H., Atlas de implantología, ed. S. Manson, 1995].

The success of a dental implant is based on the osseointegration thereof within the live tissue surrounding it. Osseointegration is defined as the direct contact between the bone and the surface of the implant, without the existence of the fibrotic capsule around the implant. In order for this to happen, there must be three steps: the first step, recruiting osteogenic cells on the surface of the implant, the formation of new bone, and finally, the remodelling of the tissue.

The osteogenic cell recruitment process is mediated by the absorption of proteins from the bloodstream in the surface of the implant, given that the adherence between the cells and the implant is carried out through these proteins. The topography, the chemical composition, and the mechanical properties of the material of the implant are key factors in the osseointegration thereof.

Despite the important progress of the last few years, aimed at the design of metal implants, the main cause of implant failure is the bad interaction between the implant and the bone due to low osseointegration levels (33.1% of the registered cases) [Implates, S.E.d., Libro Blanco de la Implantología Dental en España. 2008, Madrid: Sociedad Española de Implantes]. The osseointegration phenomenon is relate to the surface properties of the implant, the most important of which are chemical composition, hydrophilicity, and roughness [Le Guéhennec, L., et al., Dental Materials, 2007, 23(7): p. 844-854].

The degradation of implants is unadvisable, both due to the loss of structural integrity of the implant and the detachment of the products derived from its corrosion, which may cause adverse reactions in the surrounding tissue. In fact, many authors have described local concentration increases of metal traces related to the presence of an implant in the area. The corrosion of metal implants affects the environment in three different ways: electric currents affecting cell behaviour, changes in the electrochemical state (pH, O₂ concentration), and with negative effects in cell metabolism due to the presence of metal ions [Balamurugan, A., et al., Materials and Corrosion-Werkstoffe Und Korrosion, 2008, 59(11): p. 855-869]. These processes can increase the likelihood of the so-called foreign body reaction and the subsequent failure of the implant.

A solution for these materials of metallic nature to comply with the requirements demanded in terms of wear and tear and corrosion, as well as to improve osseointegration, is related to the modification of the metal surface by means of coatings.

There are different techniques to coat metal implants [Paital, S.R. and N.B. Dahotre, Materials Science and Engineering: R: Reports, 2009, 66(1-3): p. 1-70; and Liu, X., P.K. Chu, and C. Ding, Materials Science and Engineering: R: Reports, 2004, 47(3-4): p. 49-121]. There are hydroxyapatite-based, alumina-based, and titania-based coatings, as well as vitreous coatings, etc. The most widely used coatings are hydroxyapatite-type ceramic coatings, belonging to calcium phosphates, which have high bioactivity levels thanks to their similarity to the organic part of the bone.

There are different techniques to deposit ceramic coatings on the metal surface. The most widely used technique is plasma spray [Le Guéhennec, L., et al., Dental Materials, 2007, 23(7): p. 844-854; Liu, X., P.K. Chu, and C. Ding, Materials Science and Engineering: R: Reports, 2004, 47(3-4): p. 49-121]. With this technique, HAp coatings may be created [Xue, W., et al., Biomaterials, 2004, 25(3): p. 415-421] with thicknesses ranging between 50 and 200 microns [Liu, X., P.K. Chu, and C. Ding, Materials Science and Engineering: R: Reports, 2004, 47(3-4): p. 49-121]. Their main disadvantage is the low adherence between the created layer and the titanium. In addition, high temperatures are needed during the manufacturing process (close to the melting point of ceramic) and the projected HAp can decompose and form other phases with different crystallinity and a different Ca/P ratio, some of which are undesirable due to their poor *in vivo* performance. Another disadvantage is the potential alteration of the mechanical properties and resistance to corrosion due to the high temperatures reached for their application, owing to which an accelerated failure of the implant can take place [Sastre, R., S. Aza de, and J. San Román, Biomateriales, 2004]. Layers of calcium silicate (CaO - SiO₂), titanium oxide, or oxides such as zirconium or aluminium oxides, can also be obtained with this technique [Liu, X., P.K. Chu, and C. Ding, Materials Science and Engineering: R: Reports, 2004, 47(3-4): p. 49-121].

Another novel technique to increase the osseointegration of the titanium surface is biochemical modification. The objective of this technique is the modification of the surface to induce specific cell behaviour by means of the immobilization of peptides, proteins, and growth factors. The titanium is unable to anchor these molecules to its surface, due to which this technique proposes the anchoring of organosilanes, organophosphates, or photosensitive chemical products to active terminal groups (thiol-, amino-, carboxyl-, or epoxy-) capable of reacting with the biomolecules. There are different methods, such as silanization [Hoffmann, B., M. Feldmann, and G. Ziegler, Journal of Materials Chemistry, 2007, 17(38): p. 4034-4040; Xiao, S.J., et al., Journal of Materials Science-Materials in Medicine, 1997, 8(12): p. 867-872], photochemistry [Erdtmann, M., R. Keller, and H. Baumann, Biomaterials, 1994, 15(13): p. 1043-1048], or self-assembled layers (SAM's) [Mani, G., et al., Journal of Biomedical Materials Research Part B-Applied Biomaterials, 2009, 90B(2): p. 789-801]. These layers work very well in *in-vitro* cultures. However, in terms of their final application, they have the disadvantage of having coatings of a nanometric scale and there is no control on the permanence thereof on the surface of the implant after they have been inserted in the jaw.

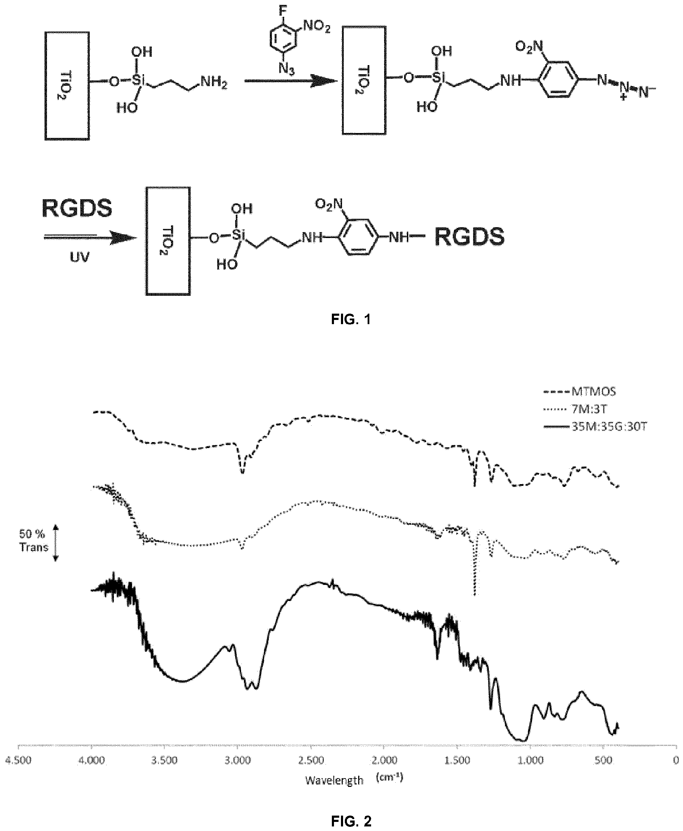

Figure 1 shows an example of silanization for the anchoring of RGD to the surface of the titanium [Hoffmann, B., M. Feldmann, and G. Ziegler, Journal of Materials Chemistry, 2007, 17(38): p. 4034-4040].

A completely innovative manner of obtaining improvements in the osseointegration of titanium implants would consist in the modification of the surface of the implant itself by means of strongly adherent coatings to the metal surface, which may be obtained by means of techniques such as sol-gel. These coatings would have thicknesses of up to 5 microns and would fulfil two fundamental objectives: in the first place, to form a surface where osteoinductive cells would tend to proliferate and mineralize with much greater intensity than the surface of the titanium, and in the second place, to serve as the release carrier for active molecules and drugs. The coatings to be formed would be hybrid, a mixture of chains, which main element is silicon, and others where carbon is the main element.

Studies about the connection mechanisms between the bone and the implant have proven that the presence of an apatite layer similar to the mineral composition of the bone favours osseointegration processes. The calcium ions and the silanol groups (Si-OH) are essential components for the formation of this biologically active layer. Due to the foregoing, the materials obtained with the sol-gel technology, based on alkoxysilanes are expected to have good properties in terms of bioactivity.

The use of implantable medical devices emplying sol-gel composition coatings as bioactive material reservoirs is disclosed in GB 2462883 A and US 2007/071789 A1.

The versatility of the sol-gel process allows controlling the degree of hydrophobic groups present in the surface, by means of the selection of the precursors used for the synthesis, and to ultimately adjust the final properties of the coating in several aspects, such as anti-corrosion properties, hydrolytic degradation, or cell viability [Zolkov, C., D. Avnir, and R. Armon, Journal of Materials Chemistry, 2004, 14(14): p. 2200-2205]. As previously explained above, the surface properties of the materials to be implanted *in vivo* are crucial for the success or failure of the biocompatibility of the prosthesis. Therefore, being able to control parameters such as hydrophilicity, degradation speed, or mechanical properties provides a powerful tool to obtain coatings with the best biocompatibility properties possible, that is to say, "tailor-made" for this application.

In addition, it has been observed that, during the hydrolytic degradation of the sol-gel, orthosilicic acid is produced in the biological fluid, by means of the reaction (ec. 1).

SiO₂ (s) + 2H₂O → Si(OH)₄ (aq) (ec. 1).

Not only these molecules in low concentrations are not toxic, but in addition they are osteoinductive [Reiner, T., S. Kababya, and I. Gotman, Journal of Materials Science-Materials in Medicine, 2008, 19(2): p. 583-589; Reffitt, D.M., et al., Bone, 2003, 32(2): p. 127-135; Hench, L.L., Bioceramics, Vol 16, M.A. Barbosa, et al., Editors, 2004, Trans Tech Publications Ltd.: Zurich-Uetikon, p. 3-6; Patel, N., et al., Journal of Materials Science-Materials in Medicine, 2002, 13(12): p. 1199-1206; Gupta, R. and A. Kumar, Biomedical Materials, 2008, 3(3)]. On the one hand, several researchers referred to in the bibliography have detected that the silicon ions favour the bioactivity of the material, thereby favouring the formation of the biomimetic hydroxyapatite layer in contact with human fluid [Patel, N., et al., Journal of Materials Science-Materials in Medicine, 2002, 13(12): p. 1199-1206; Gupta, R. and A. Kumar, Biomedical Materials, 2008, 3(3)]. On the other hand, they are capable of activating the production of type I collagen in osteoblasts and of promoting the differentiation of the same [Reffitt, D.M., et al., Bone, 2003. 32(2): p. 127-135]. Therefore, in a critical manner, the materials can be classified as biodegradable.

The present invention relates, therefore, to an organic-inorganic coating synthesised with a soft chemical method, specifically via sol-gel, which has a high percentage of success with respect to the limitations described above, that is to say, it is designed to be suitable with respect to the improvement of bioactivity and the osseointegration and osteoinduction capacity of the implant. In addition, thanks to the versatility of this manner of obtaining coatings, therapeutic agents, such as drugs, have been introduced. This coating would serve as a carrier for the release of active molecules and drugs, and would also be biodegradable. These coatings can be designed in terms of their properties based on different parameters such as chemical composition and curing treatments, as shall be described below.

### Description of the invention

The present invention relates to osteoinductive coatings applied to dental prosthesis obtained by means of a sol-gel process (as described in ES201031831). These coatings fulfil a series of properties of special interest due to their application:
- they present an economical and simple application;
- they are adherent;
- they present osteogenic capacity;
- they are resorbable;
- they are a release carrier.

The coatings are prepared based on a combination of silicon precursors. The selection of the type of precursor and the existing molar ratio between the different precursors shall depend on the characteristics required in the final implant.

The coatings are synthesized from silicon precursors: methyltrimethoxysilane, tetraethyl orthosilicate, and glycidoxypropyltrimethoxysilane. The precursor taken as base for the formation of the network is methyltrimethoxysilane.

By means of the selection of the precursors used in the process, the surface characteristics of the coating in terms of its hydrophilic properties can be controlled. This way, there are precursors having a more or less hydrophilic nature depending on the non-hydrolysable organic chain they contain. The addition of different concentrations of hydrophilic precursors, such as, for example, tetraethyl orthosilicate (TEOS), so it forms a network with the starting silicon precursor, allows varying the final properties of the coating. In a preferred manner, tetraethyl orthosilicate (TEOS) can be used in a MTMOS:TEOS molar ratio comprised between 1:0 and 4:1. In an even more preferred manner, a molar ratio of 9:1, 1:4, or 7:3 is used.

GPTMS is a silicon precursors having an organic chain with an epoxy group. The introduction of an epoxy group into the coating allows obtaining a surface with an active group to which organic agents, such as peptides or proteins, can be easily joined. The foregoing will promote cell anchoring, and therefore, osteoinduction and osseointegration.

Combinations of up to three selected silicon precursors can be made by taking MTMOS as the base precursor and by adding GPTMS and TEOS, such that, by varying the concentration thereof, a network with a higher or lower functionality due to the incorporation of the epoxy ring of the GPTMS is obtained. In addition, the hydrophilicity of the coating may be modified by means of the addition of different TEOS concentrations.

### Description of the figures

- Figure 1. Shows an example of silanization for the RGD anchoring to the surface of the titanium;
- Figure 2. FTIR spectrum of the MTMOS, 7MTMOS:3TEOS, 35MTMOS:35GPTMS:30TEOS coatings;
- Figure 3. Scheme of the structure of the network formed in the coatings;
- Figure 4. Scheme of the application procedure of the coatings to the dental implant;
- Figure 5. Chemical bond between the surface of the implant and the sol-gel coating;
- Figure 6. Compilation of the results of the adherence tests. The figure shows SEM images of the screw without coating, of the coated screw, and in the three threading areas, as well as an EDX spectrum in the severe threading area;
- Figure 7. Cell proliferation curve of human osteoblasts on titanium and osteoinductive coating obtained from MTMOS;
- Figure 8. Shows the formation of the mineralized extracellular matrix from the quantification of the calcium deposits formed by mesenchymal cells in the osteoblast differentiation process;
- Figure 9. Hydrolytic degradation curves depending on TEOS content;
- Figure 10. Left - SEM image after 1 week. Right - silicon (sol-gel-10b) and calcium (bone-10a) mapping;
- Figure 11. Thicknesses of the coating 1 week (left) and 8 weeks (right) after implantation;
- Figure 12. Weight loss curve caused by the hydrolytic degradation of the MTMOS coating in non-severe and in severe curing conditions;
- Figure 13. Reaction scheme of the amine group of the peptide and the epoxy group of the silicon precursor (GPTMS);
- Figure 14. Therapeutic agent release curve depending on the composition;
- Figure 15. Procaine release curve for a 7MTMOS:3TEOS coating with severe and non-severe curing;
- Figure 16. Hydrolytic degradation curve and procaine release curve of the 7MTMOS:3TEOS coating;
- Figure 17. Left, shows control after 1 week; right, shows 70MTMOS:30TEOS after 1 week. A greater number of active spicules and better-preserved bone marrow in comparison with the control are observed;
- Figure 18. Left, shows control after 2 weeks; right, shows 70MTMOS:30TEOS after 2 weeks. A greater number of active spicules and better-preserved bone marrow in comparison with the control are observed;
- Figure 19. State of the bone marrow 1 week after implantation. Left, control sample. Right, 70MTMOS:30 TEOS samples. The bone marrow is in a better state than in the control sample.
- Figure 20. Determination of optical density in terms of time during the formation of a mineralized extracellular matrix.

### Detailed description of the invention

The synthesis procedure of the coating object of the invention can be carried out according to the procedure described in ES201031831.

As described in said patent application, once the silicon precursor or precursors to be used in the process are selected, their dissolution is carried out in at least one solvent, of those found in the state of the art, during a first step (a), said solvent being selected such that it allows the dissolution of the silicon precursor in water, or the dissolution of two or more silicon precursors in one another. In a preferred manner, the solvent used can consist of an organic solvent, preferably selected from a group consisting of primary alcohols, secondary alcohols, or tertiary alcohols, and in an even more preferred manner, it can be selected from a group consisting of ethanol, methanol, isopropanol, and t-butanol, as well as any combination thereof. More preferably, the organic solvent used can consist of isopropanol. The selection of the solvent will have an influence on the procedure and on the type of network being generated, due to its influence in the condensation and drying process of the final coating obtained.

Likewise, in order for the network of the polymeric coating to be formed, the hydrolysis of the alkoxide groups present in the silicon precursors must take place. Said hydrolysis can take place both, in an acid medium and in a basic medium; however, the hydrolysis in an acid medium allows obtaining more condensed networks than in a basic medium, given that it favours a quick hydrolysis and the subsequent formation of greater bonds between silanols. This way, after the silicon precursor has been dissolved in the solvent, a stoichiometric amount of water at an appropriate acid pH, preferably comprised between 5 and 1, and in an even more preferred manner at a pH of 1, may be added to the dissolution. To achieve said acidity values, at least one appropriate acid such as, for example, nitric acid, may be used. The resulting mixture is then stirred for a sufficient period of time so the full hydrolysis of the alcoxide groups of the silicon precursor or starting organosilane compound takes place. In a preferred manner, the stirring time is approximately 90 minutes and cannot exceed 2 hours, with the purpose of avoiding the segregation of phases due to the partial precipitation of the organosilane compound. This way, the product obtained after step (a) consists in the dissolution of at least one hydrolyzed silicon precursor.

The procedure can likewise comprise an additional step (b) of dissolution and/or dispersion of at least one substance selected from a group consisting of an active molecule, drug, and/or peptide, as well as any combination thereof.

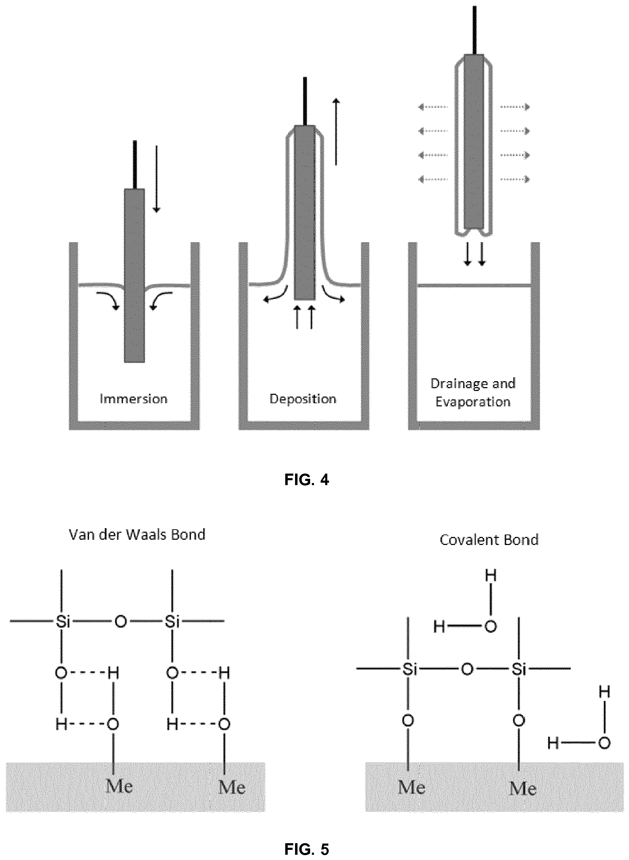

Next, in step (c) of the process, the prepared mixture is applied to the dental prosthesis in a simple and economical manner. To achieve the foregoing, the immersion technique is applied, which consists in the vertical introduction of the dental implant in the prepared mixture at a constant immersion speed, preferably comprised between 50 and 200 mm/min, for which it is possible to use any commercially available device for this purpose. Figure 4 shows a scheme of the application process of the coatings to the dental implant.

The final thickness of the coating can be comprised between 0.5 and 4 µm, a thickness of approximately 2 µm being preferred. The coating is homogenously spread throughout the surface of the implant without there being imperfections such as pores or cracks.

Next, in step (d) of the process, the coated dental prosthesis is submitted to a drying treatment with the purpose of achieving a first densification and avoiding the appearance of cracks or pores in the coating. Therefore, in a particular embodiment of the process, the drying treatment can be carried out by preferably submitting the coating to an isotherm comprised between 50 °C and 70 °C for a period of time preferably comprised between 5 and 20 min, followed by a heating ramp until the curing temperature is reached. Said thermal curing treatment leads to the total densification and condensation of the coating, and preferably can be carried out at a temperature comprised between 35°C and 140 °C for a period of time comprised between 60 and 120 minutes.

In a particularly preferred embodiment of the invention, said step (d) can comprise a first drying phase at a temperature of 50 °C for 15 min, followed by a thermal treatment or curing phase at a temperature of 140 °C for 90 minutes.

After the application of the curing treatment, the densification of the network takes place, leading to an organic-inorganic hybrid structure, as shown in the spectrums registered by means of FTIR (Figure 2), where the spectrums of the MTMOS, 7MTMOS:3TEOS, (35MTMOS:35GPTMS):30TEOS coatings are shown.

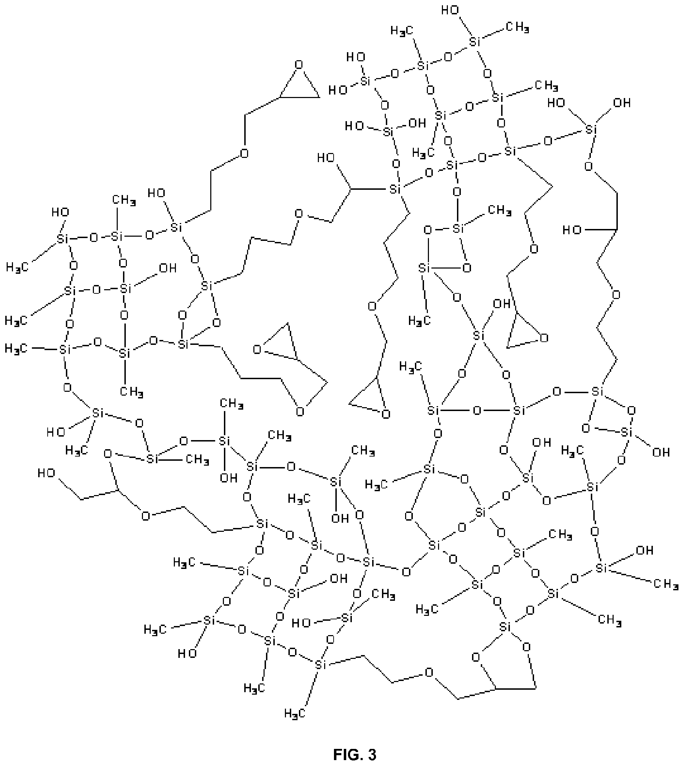

The inorganic network is polysiloxanic (Si-O-Si), and the organic network will depend on the selected precursor, as well as on its molar ratio. A scheme of the structure of the formed network, in the case of the (5MTMOS:5GPTMS):3TEOS formulation, is shown in Figure 3.

### Example 1. Determination of adherence

The coating is adhered to the surface of the implant by physical and chemical means, with the formation of covalent bonds between the titanium oxide layer and the sol-gel network. Figure 5 shows the chemical bond formed between the surface of the implant and the sol-gel coating.

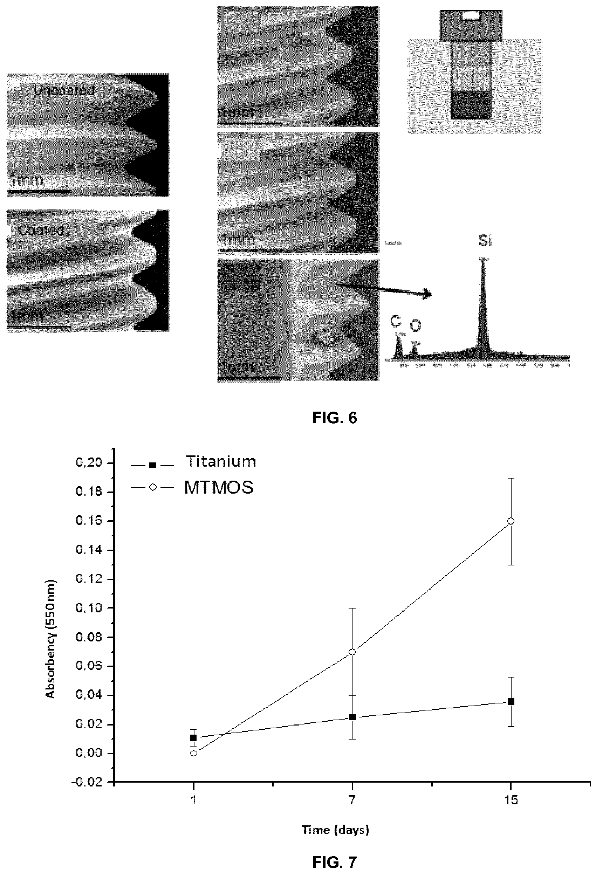

The adherence of the coatings to the dental implants, as well as the resistance to the threading process inherent to the implantation process, has been proven. To achieve the foregoing, implants coated with the different formulations have been screwed in bones with a bone density similar to the human jaw and then unscrewed. The surface finish of the implants before and after the screwing process was evaluated and the results are shown in Figure 6.

There are three threading severity areas, depending on the stress withstood by the implant when entering into contact with the bone. The presence of coating has been detected in all of them, but in higher or lower amounts depending on the area of the implant (the greater the stress severity, the lower the sol-gel amount).

### Example 2. Osteogenic capacity

The osteogenic cell recruitment capacity has been evaluated by means of human osteoblast proliferation tests on the surface of the different coatings. The results show that the MTMOS coating improves the proliferation of human osteoblasts in an effective manner in comparison with the results obtained for titanium (Figure 7).

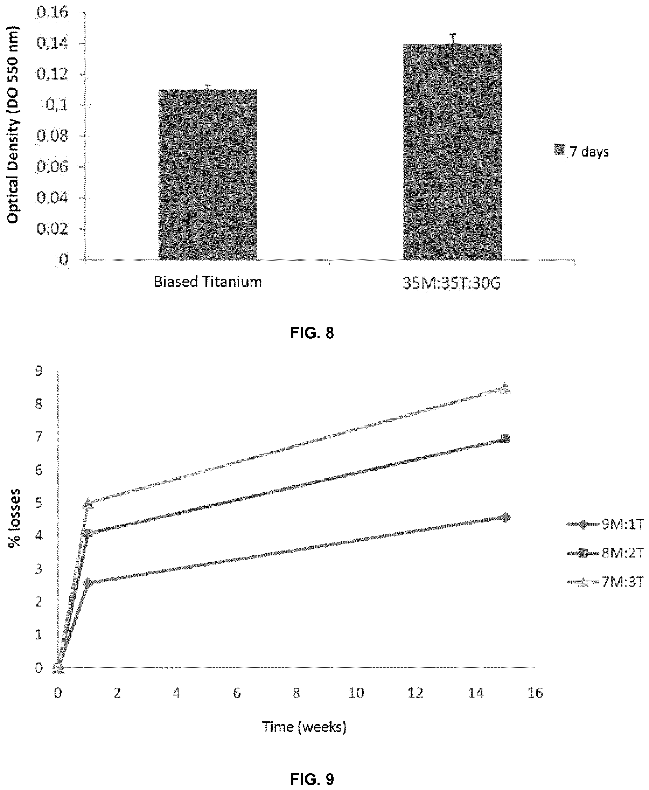

The capacity to recruit cells capable of regenerating bone tissue has been evaluated by means of mesenchymal stem cell proliferation tests. The way in which the created surface affects the osteogenic capacity of this cell type in comparison with the titanium behaviour with the customary surface treatment used in dental implantology (blasted titanium), has been evaluated. Figure 8 shows the results of the quantification of the calcium deposits formed by mesenchymal cells in the osteoblast differentiation process.

The results show how the 35MTMOS:35GPTMS:30TEOS formulation has a mineralized extracellular matrix production well above to that of commercial dental implants. Therefore, the promotion of the osteoinduction of the implant by means of the creation of the sol-gel coating has been achieved.

### Example 3. Resorption capacity - reference example

Coatings are resorbed in contact with live tissue by means of hydrolytic degradation. Degradation was evaluated by means of *in vitro* and *in vivo* tests. This way, the degradation of the coatings in contact with aqueous means can be controlled in two different ways, by means of:
(a) the composition; or
(b) the crosslinking degree of the network (as described in patent application ES201031831)

### (a) Composition

As indicated above, with the selection of the precursors used in the procedure, the surface characteristics of the coating in terms of its hydrophilic properties may be controlled.

The addition of increasing amounts of TEOS in the formulation brings about the formation of more hydrophilic networks that degrade at a greater speed. Figure 9 shows the hydrolytic degradation curves depending on TEOS content.

In addition, the resorption of the coatings *in vivo* was verified. To achieve the foregoing, the coated screws were implanted in rabbit tibia and extracted at different weeks (1, 2, 3 and 4 weeks). The non-calcified samples were embedded in methyl methacrylate (MMA) and cut with an EXAKT (EXAKT Vertriebs, Norderstedt, Germany) cutting-grinding system. The samples were analyzed with SEM and a dispersive energy X-ray spectroscopy microanalysis and mapping (SEM-EDX) were carried out.

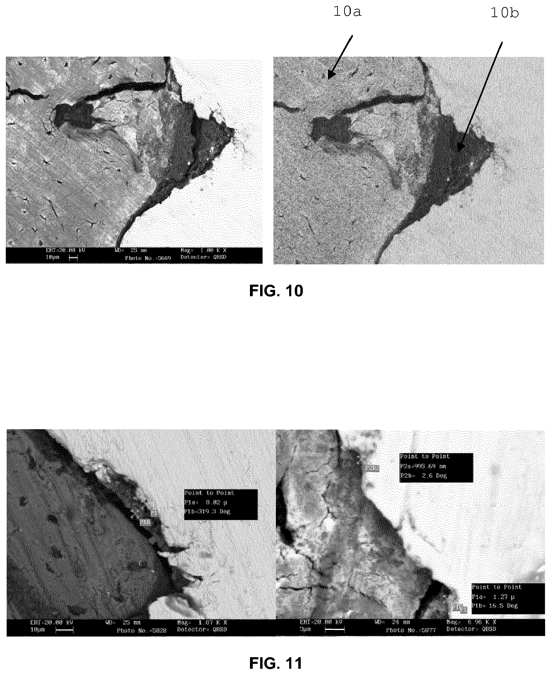

The existence of the interaction between the bone and the sol-gel coating was observed. The coating starts degrading in contact with the bone and an active interface is created, where the silicon ions disperse around the surrounding tissue. Figure 10 shows an example of the result after 1 week. On the left, there is a SEM image after 1 week and on the right, a calcium (bone - 10a) and silicon (sol-gel - 10b) mapping.

The thickness of the coatings over time was measured with the help of the SEM equipment software. It was observed the way in which the coating tends to resorb in contact with live tissue, as shown in Figure 11. The left shows the results after 1 week (with a thickness of 8 µm) and the right shows the results after 8 weeks (with a thickness of 1 µm).

### (b) Applied curing treatment (as described in patent application ES201031831).

The degradation speed of the coating may be varied with changes in the curing treatment, as described in patent application ES201031831.

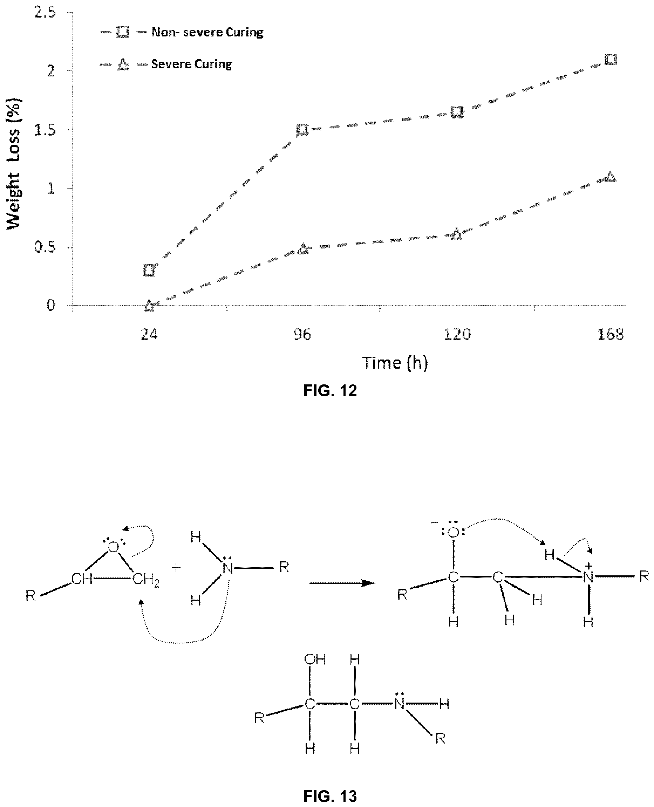

The effect of the curing treatment in the degradation speed has been analyzed *in vitro,* as shown in Figure 12. For the MTMOS coating under severe curing treatment (80 °C/2h), a slower degradation and lower material loss than in the coating with the non-severe curing treatment (80 °C/2h) were obtained.

It can be then concluded that the hydrolytic degradation speed, as well as the amount of material being degraded may be increased with the appropriate curing treatment.

### Example 4. Release carrier - reference example

Therapeutic agents or active molecules (peptides or proteins) can be incorporated to the synthesis process (as described in ES201031831). The therapeutic agent or the active molecules can be introduced into the sol-gel matrix or by means of the functionalization of the surface.

If a protein or a peptide is being introduced, the chemical anchoring of the peptide to the epoxy group of the GPTMS takes place through the amine group of the peptide, according to the process described in Figure 13.

If a model drug is being introduced, such as, for example, procaine, the release speed of the incorporated agents is once again controlled in two ways:
(a) composition
(b) applied curing treatment (as described in ES201031831)

### (a) Composition

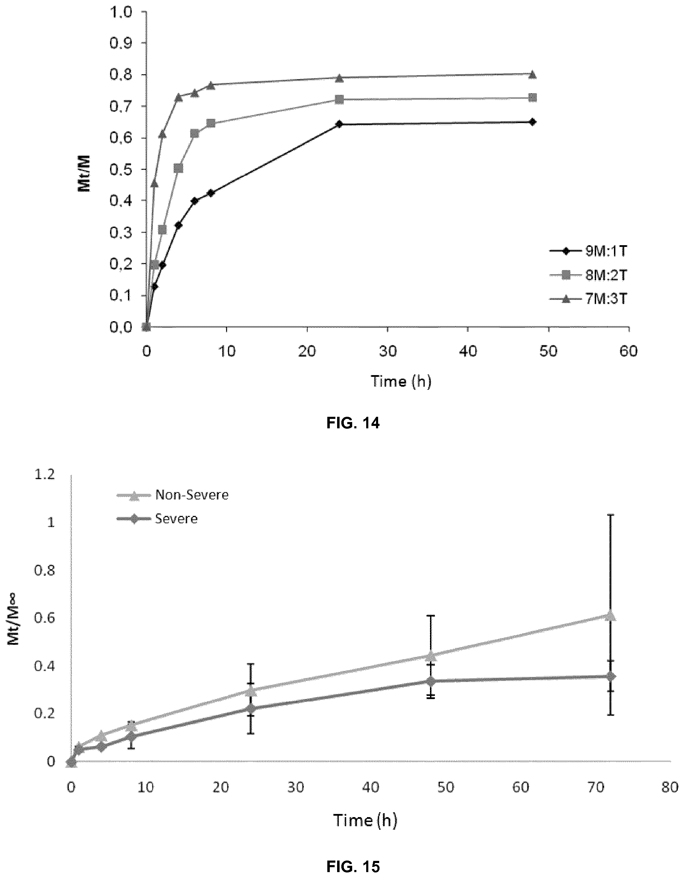

Coatings with a greater hydrophilic nature have a greater therapeutic agent release speed. Therefore, the addition of increasing amounts of TEOS in the formulation brings about the formation of more hydrophilic networks that degrade at a greater speed (Figure 14).

### (b) Applied curing treatment

Another process to vary the therapeutic agent release speed is by means of changes in the curing treatment of the network (as described in ES201031831),

Coatings with a non-severe curing treatment release at a greater speed than those with a severe curing treatment, where the network formed is denser (Figure 15).

Different degradation kinetics were obtained by controlling the curing of the network. The system in the "*severe curing*" state forms a denser network that prevents the release of the agents in its interior, the release being produced by diffusion.

### Example 5. 7MTMOS:3TEOS osteoinductive coating - reference example

This example relates to an osteoinductive coating obtained from MTMOS and TEOS precursors, with a mole ratio of 7:3 (MTMOS:TEOS).

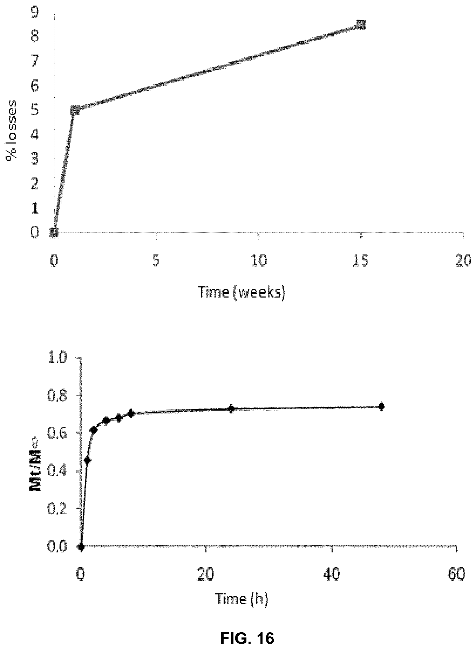

The degradation and release of therapeutic agents of the coating was evaluated *in vitro* (Figure 16).

This coating has a hydrophilic nature, and therefore, a high degradation ratio is expected, as well as a high release rate when loaded with a therapeutic agent.

In order to assess biocompatibility and the osteoinductive and resorption capacity of the sol-gel coatings developed *in vivo,* 3 repetitions of coated dental screws were implanted. Uncoated screws were used as control. The periods of study where 1, 2, 4, and 8 weeks. Non-decalcified samples where embedded in methyl methacrylate (MMA), dyed with Wheatley trichrome, and cut with the EXAKT (EXAKT Vertriebs, Norderstedt, Germany) cutting-grinding system.

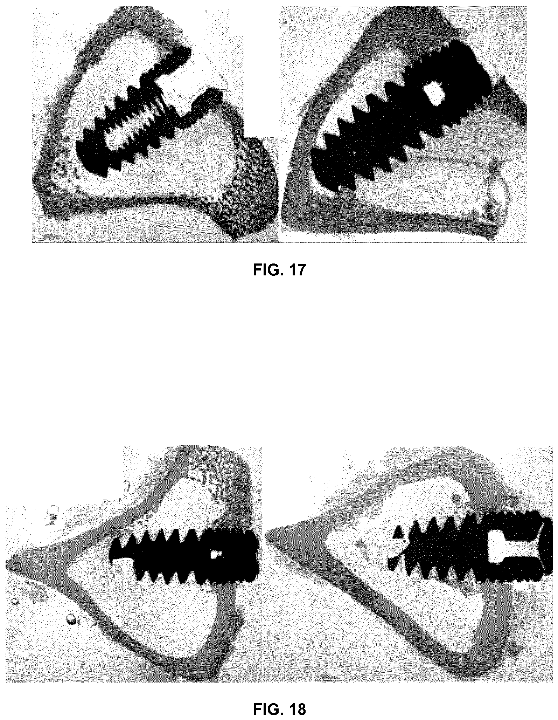

The biological response of the tissue to the coating was evaluated with optical microscopy, and it was observed a better behaviour in biocompatibility and a greater osteoblastic activation in the first periods of 1 and 2 weeks of the 70MTMOS:30TEOS formulation with respect to the control (Figures 17 and 18). On the left, the results of the control sample are shown, and on the right, the results of the 70MTMOS:30TEOS sample, after one week in Figure 17 and after two weeks in Figure 18. As shown in the figures, there are a higher number of active spicules (better-preserved bone marrow) in comparison with the control.

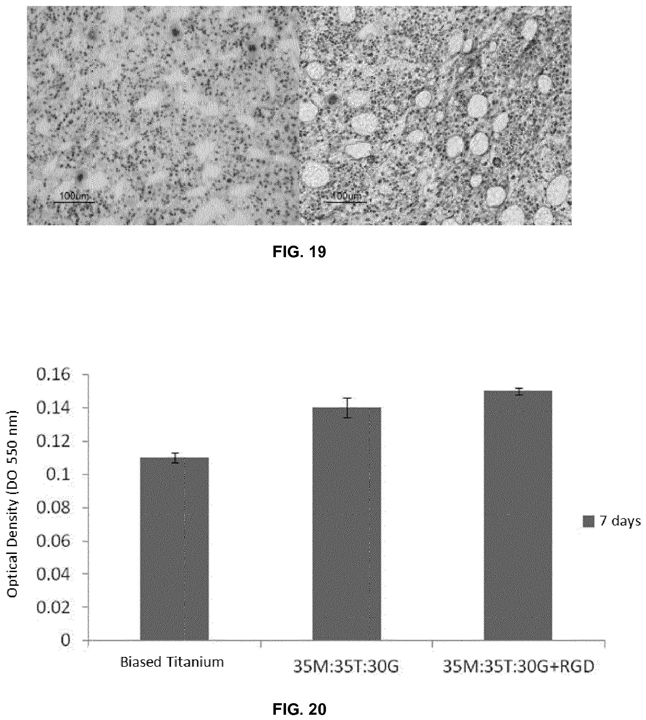

The state of the bone marrow is a good indicator of the biocompatibility of the material, given that the bone marrow is a hematopoietic organ. The preservation of the physiologic proportions between its different components and adipose tissue indicates its traumatic/post-traumatic state and its regeneration process (Figure 19). This figure shows the state of the bone marrow 1 week after implantation. On the left, the results of the control sample are shown, and on the right, the results of the 70MTMOS:30TEOS sample. As shown in the figure, in this case the bone marrow is preserved in a better state than in the control sample. Another aspect to be evaluated with respect to biocompatibility is the presence of foreign body reaction cells with respect to the biomaterial, which are cells that are found (in a low percentage) to the same extent both, in the control material and in the materials, in all the experimentation periods.

The third factor in the biocompatibility study is the morphology and evolution of the fibrous capsule formed around the biomaterial since its implantation. The formation of the fibrous capsule forms part of the physiology of the inflammation process. In the 70MTMOS:30TEOS material, the fibrous capsule formed is slightly less dense than in the control during the first week of evolution. After two weeks, both fibrous capsules are equated and suffer the same evolution until their resorption in areas with bone contact, after 4 and 8 weeks. However, in the other two materials, the density of the fibrous capsule, in addition to being higher, is never reduced and has not been resorbed after 8 weeks. In these materials, the fibrous capsule between the material and the neo-formed bone persists, preventing the osseointegration of the implant.

### Example 6. 35MTMOS:35GPTMS:30TEOS osteoinductive coating and peptides anchoring to the coating matrix

In this last example, the functionalization of the coating surface and the control of hydrophilicity were carried out, for which purpose precursors (specifically, GPTMS) with active epoxy groups capable of anchoring to peptides, were added. The TEOS precursor was added with the purpose of increasing the hydrophilic nature of the coating. The process started with the MTMOS-based white sol-gel. The existing mole ratio among the precursors is 35MTMOS:35GPTMS:30TEOS. The mixture is dissolved in isopropanol in a ratio of 1:1 by volume. Next, the addition of the amount of water at a pH=1, necessary for the hydrolysis of the alcoxides, was carried out, and the mixture was submitted to magnetic stirring for 60 min.

On the other hand, coatings with the same conditions were prepared, but doping the coating with the RGD peptide. To achieve the foregoing, the same steps were followed and RGD at 8.5% by weight with respect to silanes was added. The RGD was added as a solid (as supplied) once 30 minutes had elapsed after the addition of hydrolysis water.

The mixture obtained in this manner was left to stand for one or two hours, after which it was applied on the metal implant by immersion. To achieve the foregoing, a dip-coating device was used, by means of which the piece was submerged at a constant speed of 100 mm/min. Next, the mixture is submerged for 1 minute and is then removed at the same speed of 100 mm/min. The coated piece is then submitted to an oven drying treatment, consisting of an isotherm at 50 °C for 15 min and a heating ramp at 3 °C/min until reaching 100°C.

After the drying treatment, the piece was submitted to an oven curing treatment consisting of an isotherm at 140 °C for 90 min. This way, we obtained a sol-gel coating with an approximate thickness of 3 µm.

Next, the coatings obtained were analyzed with infrared (FTIR) to analyze if the network was correctly formed and to characterize the functional groups present in the final coating at the same time.

The osteoinduction capacity of the coatings was evaluated using *in vitro* cultures of mesenchymal cells of adipose tissue. To achieve the foregoing, the calcium deposits formed by these cells in the osteoblast differentiation process were quantified. The osteoinduction of dental implants was improved, given that the formation of the calcium deposits was higher than the one obtained for the blasted titanium implant. The incorporation of RGD slightly improves the osteoinduction capacity of the coating.

## Claims

1. Osteoinductive coating for use in dental implants, obtainable by a sol-gel process from methyltrimethoxysilane (MTMOS) as a silicon base precursor and tetraethyl orthosilicate (TEOS) as a hydrophilic silicon precursor; and at least one silicon precursor that is glycidoxypropyltrimethoxysilane, the molar ratio between the three components being (35MTMOS:35GPTMS):30TEOS.

2. The coating according to claim 1, **characterized in that** it also comprises at least a substance selected from a group consisting of an active molecule, drug, and peptide, as well as any combination thereof.

3. Process for obtaining a coating according to claim 1, **characterized in that** it comprises a step (a) of dissolution and hydrolysis of the silicon precursors, a step (c) of application of the mixture on a dental prosthesis by immersion at a constant speed of between 50 and 200 mm/min, and a step (d) of drying and curing the coating, the drying being carried out at an isotherm comprised between 50°C and 70°C for a time comprised between 5 and 20 min, and the curing at a temperature comprised between 35°C and 140 °C, for 60 to 120 minutes.

4. The process for obtaining a coating according to claim 3, **characterized in that** it comprises a step (b) following the step (a) of dissolution and hydrolysis of the silicon precursors, wherein said step (b) comprises the dissolution and/or dispersion of at least one substance selected from a group consisting of an active molecule, drug, and peptide, as well as any combination thereof.

5. The process according to any one of the claims 3 or 4, wherein the application is carried out until a thickness comprised between 0.5 and 4 µm is obtained, the coating being homogeneously spread throughout the surface of the dental prosthesis.

6. Coating according to any one of the claims 1 or 2, for use in the treatment of a deficit in bone quality and/or quantity in the area where dental prosthesis are implanted.

## Patentansprüche

1. Osteoinduktive Beschichtung zur Verwendung in Zahnimplantaten, erhältlich durch ein Sol-Gel-Verfahren aus Methyltrimethoxysilan (MTMOS) als Siliciumbasisvorstufe und Tetraethylorthosilicat (TEOS) als eine hydrophile Siliciumvorstufe; und wenigstens einer Siliciumvorstufe, welche Glycidoxypropyltrimethoxysilan ist, wobei das Molverhältnis zwischen den drei Komponenten (35MTMOS:35GPTMS):30TEOS beträgt.

2. Beschichtung gemäß Anspruch 1, **dadurch gekennzeichnet, dass** sie auch wenigstens eine Substanz, ausgewählt aus einer Gruppe bestehend aus einem aktiven Molekül, Arzneimittel und Peptid sowie einer beliebigen Kombination davon, umfasst.

3. Verfahren zum Erhalten einer Beschichtung gemäß Anspruch 1, **dadurch gekennzeichnet, dass** es einen Schritt (a) der Auflösung und Hydrolyse der Siliciumvorstufen, einen Schritt (c) des Aufbringens der Mischung auf eine Zahnprothese durch Eintauchen mit einer konstanten Geschwindigkeit zwischen 50 und 200 mm/min und einen Schritt (d) des Trocknens und Härtens der Beschichtung umfasst, wobei das Trocknen bei einer Isotherme zwischen 50°C und 70°C für eine Dauer zwischen 5 und 20 min durchgeführt wird und das Härten bei einer Temperatur zwischen 35°C und 140°C für 60 bis 120 Minuten durchgeführt wird.

4. Verfahren zum Erhalten einer Beschichtung gemäß Anspruch 3, **dadurch gekennzeichnet, dass** es einen Schritt (b) im Anschluss an den Schritt (a) der Auflösung und Hydrolyse der Siliciumvorstufen umfasst, wobei der Schritt (b) die Auflösung und/oder Dispergierung von wenigstens einer Substanz, ausgewählt aus einer Gruppe bestehend aus einem aktiven Molekül, Arzneimittel und Peptid sowie einer beliebigen Kombination davon, umfasst.

5. Verfahren gemäß einem der Ansprüche 3 oder 4, wobei das Aufbringen ausgeführt wird, bis eine Dicke zwischen 0,5 und 4 µm erhalten wird, wobei die Beschichtung homogen auf der ganzen Oberfläche der Zahnprothese ausgebreitet wird.

6. Beschichtung gemäß einem der Ansprüche 1 oder 2 zur Verwendung bei der Behandlung eines Mangels der Knochenqualität und/oder -quantität in dem Bereich, wo Zahnprothesen implantiert werden.

## Revendications

1. Revêtement ostéoinducteur destiné à être utilisé dans des implants dentaires, pouvant être obtenu par un procédé sol-gel à partir de méthyltriméthoxysilane (MTMOS) en tant que précurseur de base silicium et d'orthosilicate de tétraéthyle (TEOS) en tant que précurseur de silicium hydrophile ; et au moins un précurseur de silicium qui est le glycidoxypropyltriméthoxysilane, le rapport molaire entre les trois composants étant (35MTMOS:35GPTMS):30TEOS.

2. Revêtement selon la revendication 1, **caractérisé en ce qu'**il comprend également au moins une substance sélectionnée dans un groupe consistant en une molécule active, un médicament, et un peptide, ainsi qu'une quelconque combinaison de ceux-ci.

3. Procédé pour obtenir un revêtement selon la revendication 1, **caractérisé en ce qu'**il comprend une étape (a) de dissolution et d'hydrolyse des précurseurs de silicium, une étape (c) d'application du mélange sur une prothèse dentaire par une immersion à une vitesse constante comprise entre 50 et 200 mm/min, et une étape (d) de séchage et de durcissement du revêtement, le séchage étant réalisé à un isotherme compris entre 50 °C et 70 °C pendant une durée comprise entre 5 et 20 min, et le durcissement à une température comprise entre 35 °C et 140 °C, pendant 60 à 120 minutes.

4. Procédé pour obtenir un revêtement selon la revendication 3, **caractérisé en ce qu'**il comprend une étape (b) après l'étape (a) de dissolution et d'hydrolyse des précurseurs de silicium, où ladite étape (b) comprend la dissolution et/ou la dispersion d'au moins une substance sélectionnée dans un groupe consistant en une molécule active, un médicament, et un peptide, ainsi qu'une quelconque combinaison de ceux-ci.

5. Procédé selon l'une quelconque des revendications 3 ou 4, dans lequel l'application est réalisée jusqu'à ce qu'une épaisseur comprise entre 0,5 et 4 µm soit obtenue, le revêtement étant enduit de manière homogène sur toute la surface de la prothèse dentaire.

6. Revêtement selon l'une quelconque des revendications 1 ou 2, destiné à être utilisé dans le traitement d'un déficit de qualité et/ou de quantité osseuse dans la région où la prothèse dentaire est implantée.
